# FASCICULE DE BREVET EUROPEEN

(11) **EP 0 694 296 B1**
(45) Date de publication et mention de la délivrance du brevet: **17.11.1999**
(21) Numéro de dépôt: 95401719.0
(22) Date de dépôt: 19.07.1995
(51) Int. Cl.: A61F 5/37

(54) **Gilet de contention pour l'épaule, la ceinture scapulaire et le membre supérieur**
Weste zur Unterstützung von Schulter Schultergürtel und der oberen Gliedmasse
Supporting vest for the shoulder, the girdle shoulder and the upper member

(30) Priorité: 19.07.1994 FR 9408917
(43) Date de publication de la demande: 31.01.1996
(73) Titulaire: PETERS, 93003 Bobigny Cédex (FR)
(72) Inventeur: Mandelbaum, Alain, F-76290 Montivilliers (FR)
(74) Mandataire: Phélip, Bruno

(56) Documents cités:
- WO-A-82/03767
- FR-A- 2 624 003
- GB-A- 2 181 952
- US-A- 2 344 844
- US-A- 2 560 243
- US-A- 3 559 640
- US-A- 4 878 490

## Description

La présente invention concerne un gilet orthopédique de soutien et de contention pour le traitement des traumatismes et des interventions chirurgicales de l'épaule, de la ceinture scapulaire et du membre supérieur.

De nombreux dispositifs existent déjà pour soutenir et immobiliser cette partie du corps. On peut notamment citer les écharpes de soutien simple qui permettent le soutien de l'avant-bras mais n'immobilisent, ni le bras, ni l'épaule. Il existe aussi des manchons de soutien de l'avant-bras, complétés par une bande de contention du bras, qui enserre le thorax et maintient le coude au corps sans recouvrir, ni immobiliser efficacement l'épaule traumatisée.

Des bandages élastiques, dits du type Dujarrier, sont également utilisés. Ils sont réalisés avec des bandes élastiques, immobilisant l'avant-bras, le bras et l'épaule, en enserrant aussi le thorax. Ces bandages nécessitent des connaissances appropriées pour les effectuer. De plus, ils ne sont pas solides car les bandes se relâchent, ce qui remet en cause le principe de l'immobilisation et donc l'efficacité du traitement. Ils nécessitent d'être remis en place par des personnes expérimentées. Par ailleurs, ils ne permettent pas du tout de réaliser les soins d'hygiène indispensables au patient.

On connaît par US-3,559,640 un gilet symétrique reposant sur les deux épaules et fermant sur le devant par une fermeture à glissière portée par deux panneaux hémithoraciques.

Le document US-A-2 549 703 décrit un gilet orthopédique de soutien et de contention de l'épaule, de la ceinture scapulaire et du membre supérieur qui est essentiellement composé de quatre panneaux.

Un premier panneau enserre la partie avant du thorax, située du côté du bras non malade, un deuxième panneau est destiné à enserrer la partie avant du thorax, située du côté du bras malade ainsi que la partie axillaire et la partie antérieure de l'épaule et du bras malade. Ce deuxième panneau se prolonge latéralement par un troisième panneau qui est destiné à enserrer la partie postérieure de l'épaule et du bras et qui se raccorde au premier panneau. Le deuxième panneau est également prolongé, à sa partie inférieure, par un quatrième panneau qui sert de reposoir à l'avant-bras. Les deuxième et quatrième panneaux sont équipés de moyens de fermeture et de serrage permettant au gilet d'être indifféremment utilisé à gauche ou à droite par simple retournement.

Un tel gilet présente de nombreux inconvénients. En effet, il ne peut pas être placé, ni réajusté par le patient seul, notamment parce que le bras valide doit être passé dans une ouverture étroite et que les moyens de fermeture et de serrage sont constitués par des noeuds. De plus, de tels moyens n'assurent pas, dans le temps, un réglage et un maintien uniforme sur toute la longueur de l'avant-bras.

Le document GB-1 291 780 décrit également un gilet de contention mais qui est conçu pour l'immobilisation et le soutien de deux bras simultanément. Il enserre le thorax et les deux épaules. Il peut également être utilisé pour un seul bras malade et dans ce cas, le bras valide est passé dans un orifice prévu à cet effet.

Ce gilet présente également des inconvénients car l'orifice prévu pour le bras valide est relativement étroit et la mise en place du gilet est donc difficile. De plus, une patte de serrage est prévue pour immobiliser et bloquer un coude mais ceci empêche d'exercer une surveillance locale.

Le document US-2 344 844 concerne un gilet permettant le soutien du bras, en cas de fracture de l'avant-bras. Il est constitué de trois panneaux: un panneau avant et un panneau arrière reliés au niveau des épaules, pour présenter la forme d'un gilet et reliés également sur les côtés, par des pattes de tissu; le panneau avant se prolongeant, à sa partie inférieure, par un troisième panneau qui sert de reposoir à l'avant-bras.

Un gilet de ce type ne convient pas pour le traitement des traumatismes de l'épaule puisqu'il ne peut pas assurer la contention de l'épaule.

On peut encore citer des gilets de contention de forme identique au bandage de Dujarrier, donc enserrant le thorax, et comportant une fermeture dorsale par fixation auto-agrippante. De tels gilets sont notamment décrits dans le brevet EP-A-0 077 345.

Ces gilets, qui sont formés d'une succession de panneaux rectangulaires, ne peuvent être mis en place que par un professionnel. Le patient ne peut pas enlever seul un tel gilet pour pratiquer des soins d'hygiène, ni le remettre seul, puisque le gilet doit être fermé dans le dos. Par ailleurs, la fermeture est réalisée en croisant deux panneaux. Le gilet présente donc, dans le dos, deux épaisseurs de tissu, ce qui est inconfortable pour les malades alités. Le patient a impérativement besoin d'une aide extérieure ayant la maîtrise de la pose de ce produit car il est fréquent de constater des erreurs de positionnement des panneaux dorsaux, qui, croisés dans le mauvais sens, entraînent l'application d'une partie de la fixation agrippante sur la peau, sans protection aucune. Des lésions cutanées peuvent en résulter. On constate également que le gilet ne prend appui que sur l'épaule malade et que les sollicitations y sont donc concentrées. Enfin, le gilet enserre la cage thoracique et la compresse, ce qui peut gêner la respiration du patient.

La présente invention a pour objet de pallier ces inconvénients en proposant un nouveau gilet de soutien et de contention destiné au traitement des traumatismes de l'épaule et pouvant être mis en place facilement, sans apprentissage d'une technique compliquée. Le patient peut l'ôter, notamment pour faire des soins d'hygiène et le remettre en place seul. Le gilet s'enfile aisément par la tête grâce à un orifice ménagé et s'ajuste avec beaucoup de simplicité. De plus, le gilet selon l'invention est conçu pour prendre appui sur les deux épaules, ce qui permet de mieux répartir les sollicitations. Enfin, le gilet est conçu pour être réversible.

L'invention concerne donc un gilet orthopédique de soutien et de contention de l'épaule, de la ceinture scapulaire et du membre supérieur d'un patient, ledit gilet fermant sur l'avant et étant formé de quatre panneaux :
- un premier panneau antérieur recouvrant la partie antérieure du thorax et des deux épaules et la partie antérieure du bras à soutenir, ledit premier panneau se prolongeant à l'extrémité opposée aux épaules par un deuxième panneau antérieur destiné à servir de reposoir à l'avant bras à soutenir, le premier panneau étant délimité par une ligne sensiblement rectiligne qui se raccorde au deuxième panneau et qui se prolonge, du côté opposé au deuxième panneau et de façon successive, par une première courbe destinée à être placée sur l'épaule et une deuxième courbe concave au niveau d'une ouverture ménagée entre le premier et un troisième panneau pour le passage de la tête du patient, ladite deuxième courbe se prolonge par une troisième courbe convexe descendante qui se raccorde au deuxième panneau, à l'opposé de ladite ligne;
   un troisième panneau postérieur recouvrant la partie postérieure du thorax et des deux épaules et la partie postérieure du bras à soutenir, ledit troisième panneau se prolongeant à l'extrémité opposée aux épaules du côté opposé au bras à soutenir par un quatrième panneau destiné à assurer une contention et une fermeture sur l'avant dudit gilet ;
   le premier panneau et le troisième panneau se rejoignant au niveau des épaules et le long du bras à soutenir.

Le gilet selon l'invention est également caractérisé par les caractéristiques techniques suivantes, prises isolément ou en combinaison:
- le premier panneau est délimité par une ligne sensiblement rectiligne qui se raccorde au deuxième panneau et qui se prolonge, du côté opposé au deuxième panneau et de façon successive, par une première courbe destinée à être placée sur l'épaule et une deuxième courbe concave au niveau de l'ouverture, laquelle se prolonge par une troisième courbe convexe descendante qui se raccorde au deuxième panneau, à l'opposé de ladite ligne.
- le gilet est ouvert au niveau de la première courbe, des moyens de serrage et de fixation étant prévus pour assembler lesdits premier et troisième panneaux,
- lesdits moyens de serrage et de fixation sont portés par une patte qui est dans le prolongement du premier panneau ou du troisième panneau,
- le deuxième panneau présente une forme générale en demi-lune.
- le deuxième panneau porte, à son extrémité libre, des moyens de serrage et de fixation.
- le deuxième panneau comporte latéralement et du côté opposé au bras à soutenir, une courbe concave permettant le passage de la main du patient hors du gilet.
- le deuxième panneau comporte latéralement et du côté du bras à soutenir, une courbe convexe permettant de laisser libre le coude du patient.
- les premier et troisième panneaux sont solidaires au niveau de la troisième courbe.
- le troisième panneau comporte une courbe concave, en regard de la deuxième courbe concave, lesdites courbes définissant l'ouverture.
- le quatrième panneau présente une forme allongée, sensiblement rectangulaire et comporte, à son extrémité libre, des moyens de serrage et de fixation.
- lesdits moyens de serrage et de fixation sont prévus sur les deux faces du gilet, de façon à ce qu'il soit réversible.
- le gilet comporte au moins une bande amovible de forme allongée, sensiblement rectangulaire, qui est destinée à allonger le quatrième panneau et/ou à être fixée sur les premier et troisième panneaux avant la mise en place du deuxième panneau et sous le bras malade et/ou une fois le gilet mis en place, à être fixé sur les deuxième et troisième panneaux et sur le bras malade.
- la bande amovible comporte, à deux extrémités libres opposées, des moyens d'attache.
- le gilet comporte également une languette amovible pour le soutien du pouce qui forme une boucle et comporte des moyens d'attache, ladite languette étant destinée à être fixée sur ledit deuxième panneau.
- le gilet est constitué en une matière autoagrippante.
- le gilet comporte des moyens destinés à coopérer avec lesdits moyens de serrage et de fixation et lesdits moyens d'attache.

La présente invention sera mieux comprise et d'autres buts, avantages et caractéristiques de celle-ci seront mieux compris à la lecture de la description qui suit, faite en référence aux dessins annexés sur lesquels:
- la figure 1 représente une vue en plan du gilet selon l'invention,
- la figure 2 représente une vue de face d'un patient ayant enfilé le gilet,
- la figure 3 représente une vue de derrière du patient de la figure 2,
- les figures 4 à 6 représentent des étapes de mise en place du gilet de la figure 1,
- la figure 7 représente une vue en plan d'une variante de réalisation du gilet selon l'invention et
- la figure 8 représente le gilet selon l'invention, conforme à la variante de la figure 7, une fois mis en place sur un patient.

Les éléments communs aux différentes figures seront désignés par les mêmes références.

On désigne par les références 1, 2, 3 et 4 les quatre panneaux du gilet qui sont solidaires, par la référence 5 un panneau supplémentaire amovible destiné notamment au maintien de l'avant-bras et par la référence 6, une languette additionnelle servant de support pour le pouce du patient. Comme on le verra dans la suite de la description, seuls les quatre panneaux 1 à 4 sont indispensables pour constituer le gilet.

Le premier panneau 1 est destiné à recouvrir la partie antérieure du thorax, des deux épaules et du bras à soutenir. Il se prolonge latéralement, à sa partie inférieure, par un deuxième panneau 2 qui est destiné à servir de reposoir pour l'avant-bras du patient.

Ce deuxième panneau 2 présente une forme générale de demi-lune se prolongeant, à son extrémité libre, par un panneau 7 de forme sensiblement rectangulaire supportant des moyens de serrage et de fixation 20. Le deuxième panneau est délimité latéralement par deux courbes 8 et 9. La courbe 8 présente une concavité relativement importante au niveau de son raccordement avec le panneau 7 afin de permettre le passage de la main du patient, hors du gilet. Cette disposition favorise l'autonomie du patient. De plus, la forme de la courbe 9 est choisie de façon à ce que le coude du patient soit libre.

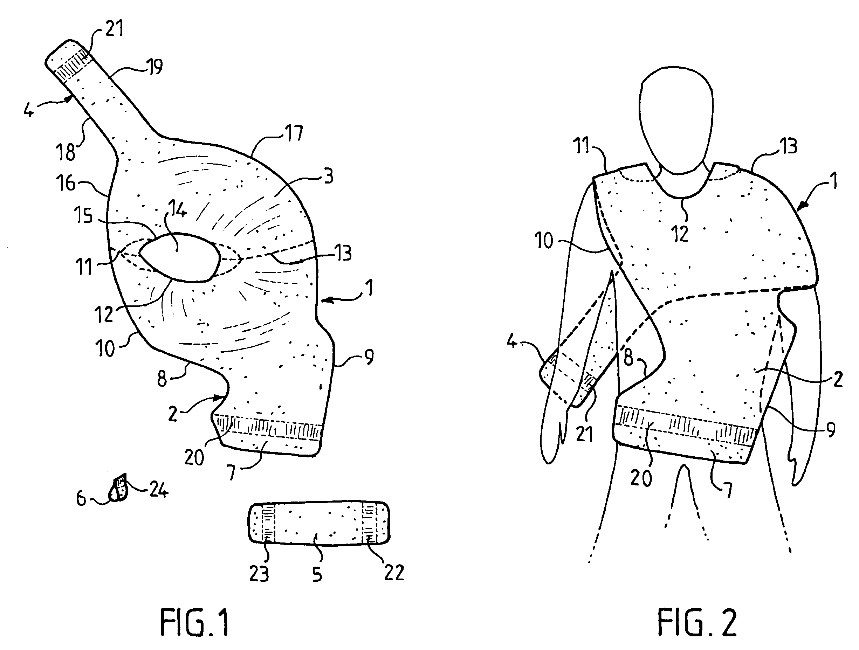

Le premier panneau 1, tel que représenté à la figure 1, est délimité tout d'abord par une ligne 10 dite supérieure. C'est une courbe sensiblement rectiligne qui, de préférence, s'infléchit légèrement vers l'intérieur du panneau au niveau de son raccordement avec le deuxième panneau 2 et avec le troisième panneau 3.

Sur le côté opposé au deuxième panneau 2, le premier panneau est délimité par trois courbes successives 11, 12 et 13. La première courbe 11 est sensiblement à angle droit avec la ligne supérieure 10. Elle est destinée à être placée au niveau de la partie supérieure de l'épaule non malade du patient. Cette première courbe 11 se prolonge par une deuxième courbe 12 concave. Comme on le verra par la suite, cette deuxième courbe contribue à définir une ouverture 14 pour le passage de la tête du patient.

La deuxième courbe 12 se prolonge elle-même par une troisième courbe 13 convexe descendante qui se raccorde au deuxième panneau 2, à l'opposé de la ligne 10.

Le troisième panneau 3 est rendu solidaire du premier panneau 1, au niveau des première et troisième courbes 11 et 13 et présente donc, à l'endroit de son assemblage avec le premier panneau, un profil identique. L'assemblage peut être effectué par tout moyen approprié et notamment par couture.

Entre les bords d'assemblage, le troisième panneau 3 comprend une courbe concave 15. Les courbes concaves 12 et 15 définissent donc l'ouverture 14 pour la tête du patient. De préférence, on choisit des formes différentes pour ces deux courbes afin de pouvoir facilement distinguer la partie antérieure du gilet, constituée par le premier panneau, de la partie postérieure, constituée par le troisième panneau. En particulier, la courbe concave 12 réalisée sur le premier panneau 1 peut prendre la forme d'une échancrure en V.

Le troisième panneau 3 est donc destiné à recouvrir la partie postérieure du thorax, des deux épaules et du bras à soutenir et plus particulièrement, les deux omoplates.

Ce troisième panneau est délimité par une courbe dite supérieure 16 qui est dans le prolongement de la courbe supérieure 10 du premier panneau 1.

Ce panneau 3 se prolonge, du côté opposé au premier panneau et au bras à soutenir, par un quatrième panneau 4.

Comme indiqué pour la courbe supérieure 10 du premier panneau, la courbe supérieure 16 du troisième panneau est de préférence légèrement convexe, c'est-à-dire qu'elle s'infléchit légèrement vers l'intérieur du panneau au niveau de son raccordement avec le premier panneau 1 et avec le quatrième panneau 4.

A sa partie inférieure, le troisième panneau 3 est délimité par une courbe 17 qui est une ligne en biais venant se raccorder au quatrième panneau. De préférence, la courbe 17 est légèrement convexe pour mieux épouser le dos du patient.

Le quatrième panneau 4 présente une forme allongée sensiblement rectangulaire délimitée par une ligne supérieure 18 et une ligne inférieure 19, la ligne supérieure 18 étant sensiblement à angle droit avec la courbe supérieure 16 du troisième panneau et la ligne inférieure 19 étant sensiblement à angle droit avec la courbe inférieure 17 du panneau 3.

Du côté de l'extrémité libre du quatrième panneau 4, sont prévus des moyens de serrage et de fixation 21.

On peut noter que les moyens de serrage et de fixation 20 et 21 prévus sur les deuxième et quatrième panneaux sont disposés, de préférence, sur les deux faces du corps du gilet, de façon à ce que ce dernier soit réversible et puisse être utilisé indifféremment à droite ou à gauche par simple retournement.

La bande 5 amovible est prévue, comme cela sera décrit par la suite, pour assurer le maintien de l'avant-bras. Elle est de forme allongée, sensiblement rectangulaire et comporte, au niveau des extrémités de ses petits côtés, des moyens d'attache 22 et 23. Elle peut également être utilisée pour rallonger le quatrième panneau 4.

La languette 6 amovible est constituée d'une bande étroite formant une boucle et comportant également des moyens d'attache 24. Cette languette est destinée à être fixée sur le deuxième panneau 2 pour servir de reposoir au pouce.

L'ensemble du gilet est constitué en un matériau jouant le rôle de bandage et permettant une aération satisfaisante pour limiter les problèmes de sudation. De préférence, le matériau est choisi de façon à être "auto-agrippant" de façon à permettre directement l'accrochage des moyens de serrage et de fixation et des moyens d'attache sur le corps du gilet, qui sont euxmêmes réalisés en un matériau du type Velcro®. Dans le cas où le matériau du gilet n'est pas auto-agrippant, il convient de prévoir, sur les panneaux, des moyens de serrage et de fixation et des moyens d'attache, destinés à coopérer avec les moyens 20 à 24 et qui peuvent notamment être constitués par une bande correspondante du type Velcro®. Ces moyens ne sont pas représentés sur les figures.

On peut noter qu'un tel matériau auto-agrippant est bien connu dans l'état de la technique, notamment dans des applications médicales. Ainsi, il est courant d'utiliser des matériaux de ce type pour réaliser des attelles de genou et des gilets pour le soutien d'un bras.

La mise en place du gilet va maintenant être décrite en référence aux figures 2 à 6.

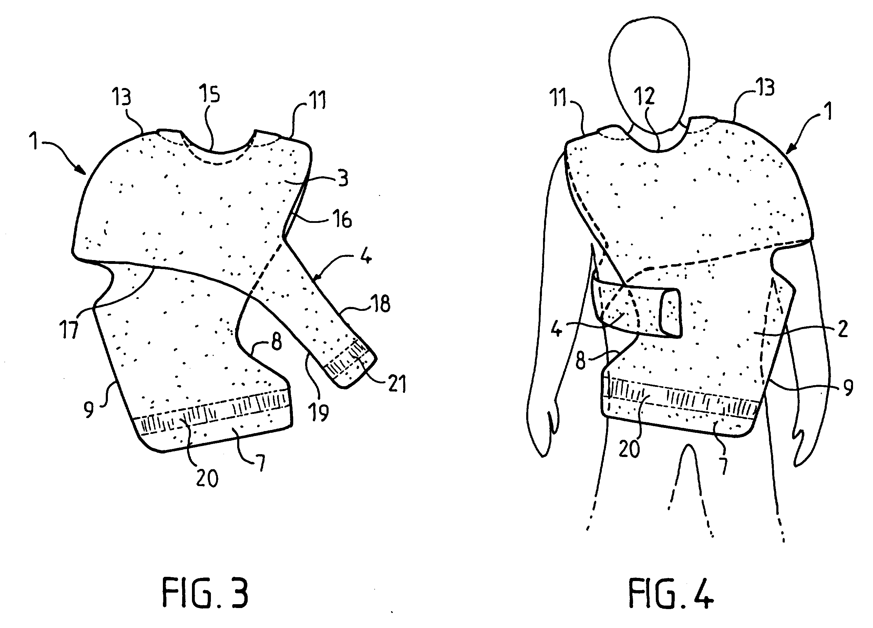

Le gilet est tout d'abord enfilé par la tête du patient pour se trouver dans la position illustrée aux figures 2 et 3. Comme indiqué précédemment, la courbe concave 12 située sur le premier panneau présente, de préférence, une forme différente de la courbe concave 15, située sur le troisième panneau. Le patient peut ainsi facilement distinguer la partie antérieure du gilet de la partie postérieure. La distinction entre la partie antérieure et la partie postérieure du gilet peut également être facilitée en réalisant chacune de ces parties en des tissus de couleur différente.

On peut noter que le patient peut enfiler seul le gilet avec sa main et son bras valides.

Une fois le gilet enfilé, le patient saisit avec sa main valide le quatrième panneau 4, situé du côté postérieur et à sa portée, et l'applique, au niveau de son extrémité libre, sur le premier panneau 1, placé sur la partie antérieure du thorax (figure 4). Les moyens de fixation et de serrage 21 coopèrent directement avec le tissu constituant le gilet et sont fixés sur le premier panneau par simple pression. Le quatrième panneau 4 se trouve alors dans la position illustrée à la figure 4. Ce panneau 4 est dans le prolongement du troisième panneau 3. Il permet donc de le fixer par rapport au premier panneau 1 et d'appliquer fermement le gilet contre le corps du patient. On comprend que la position du panneau 4 est ajustée pour régler le serrage de façon appropriée.

La bande 5 peut être utilisée lors de la mise en place du quatrième panneau 4. En effet, cette bande peut servir à rallonger le quatrième panneau si elle est rendue solidaire de celui-ci par l'intermédiaire des moyens de serrage et de fixation 21 et/ou des moyens d'attache 22 ou 23. Cette disposition permet de fermer le gilet lorsque celui-ci est destiné à des personnes corpulentes.

On peut encore noter que cette deuxième étape peut être réalisée par le patient seul, puisque la mise en place du quatrième panneau ne sollicite pas la partie du corps qui est malade.

Cette deuxième étape comporte une dernière opération qui est facultative.

Elle consiste à utiliser la bande 5 pour plaquer le gilet contre le corps du patient. A cet effet, la bande 5 est placée sous l'aisselle du patient, du côté du bras malade, pour solidariser les premier et troisième panneaux 1 et 3. La bande 5 est ainsi placée sous le bras malade.

Cette dernière opération est avantageusement réalisée lorsque le gilet est destiné à un patient alité car elle permet d'éviter que le gilet ne remonte.

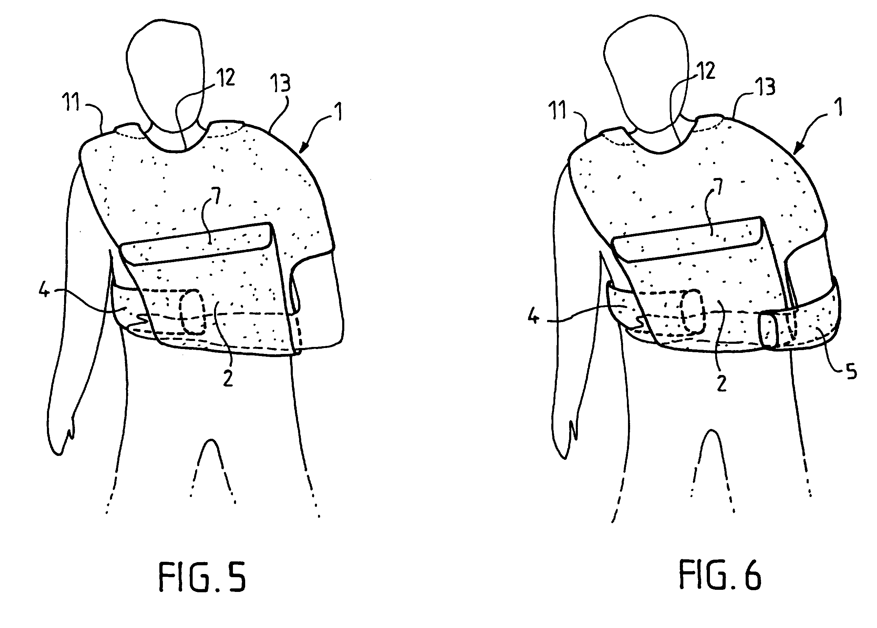

La troisième étape consiste à rabattre le deuxième panneau 2 sur le premier panneau 1 et à le fixer sur ce dernier grâce aux moyens de fixation et de serrage 20, comme cela est illustré à la figure 5.

Au cours de cette étape, l'avant-bras du patient situé du côté malade est, de préférence, replié selon un angle d'environ 90°. Dans cette position de l'avant-bras, le deuxième panneau est fixé sur le premier panneau de façon à placer l'avant-bras suivant l'angle souhaité. En effet, l'extrémité libre du panneau 7 peut être sensiblement horizontale ou encore former un angle déterminé par rapport à la ligne des épaules. Ceci peut être utile pour le traitement du membre blessé.

Au cours de cette étape, on peut également mettre en place la languette 6, si cela s'avère nécessaire. Dans ce cas, la languette 6 est placée sur le deuxième panneau 2 et plus particulièrement, sur la face qui est destinée à être en regard du premier panneau. La position de la languette est déterminée en fonction du patient. La languette est fixée sur le deuxième panneau par l'intermédiaire des moyens d'attache 24. Le pouce du patient est passé à travers la boucle formée par la languette 6 qui sert alors de reposoir et permet d'éviter que la main ne tombe. Ceci contribue donc au confort du malade.

La dernière étape est facultative et consiste à mettre en place la bande 5 qui dans ce cas, a une fonction de maintien.

La bande 5 est accrochée, par l'intermédiaire des moyens d'attache 22 et 23, à la fois sur le deuxième panneau 2 et sur le troisième panneau 3. Elle est donc placée au niveau de la tête humérale ou au niveau du coude, légèrement en biais par rapport au bras malade 4.

Dans cette position, la bande 5 permet de renforcer le maintien au niveau de l'épaule ou du coude, en cas de foyer de fracture à ce niveau. On peut encore noter que le patient peut positionner seul cette bande, grâce à sa main valide.

On peut noter que la bande 5 peut remplir trois fonctions différentes : pour rallonger le panneau 4, serrer le gilet ou renforcer le maintien au niveau de l'épaule ou du coude.

Ces trois fonctions peuvent être remplies simultanément, un nombre adéquat de bandes 5 étant alors utilisé.

Le gilet selon l'invention peut être prévu en plusieurs tailles différentes pour s'adapter aux différentes morphologies.

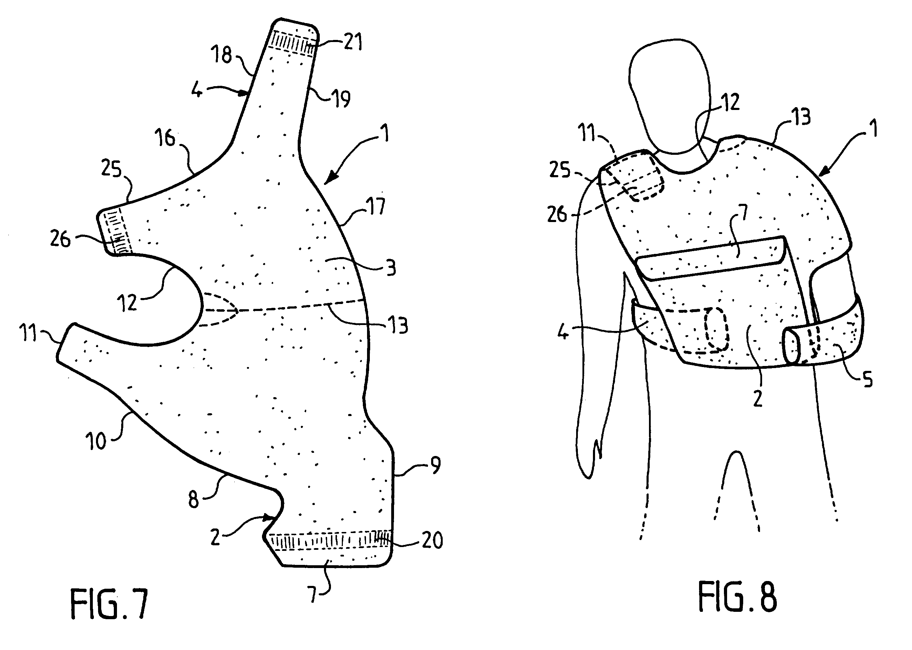

En référence aux figures 7 et 8, une variante de réalisation du gilet selon l'invention va être décrite.

Au niveau de la première courbe 11 destinée à être placée au niveau de la partie supérieure de l'épaule non malade, les premier et troisième panneaux 1 et 3 ne sont pas assemblés de manière fixe.

Le gilet est ouvert au niveau de cette première courbe 11 et des moyens de serrage et de fixation 26 appropriés sont prévus, pour assembler les premier et troisième panneaux 1 et 3, lorsque le gilet est mis en place.

Ces moyens peuvent notamment être portés par une patte 25 qui est par exemple dans le prolongement du troisième panneau 3, comme cela est illustré sur les figures 7 et 8. Elle peut également se trouver dans le prolongement du premier panneau 1.

Cette variante de réalisation présente plusieurs avantages. Elle permet tout d'abord de mettre le gilet sensiblement à plat, comme l'illustre la figure 7. Dans cette position, le gilet peut être facilement mis en place sur un patient qui est allongé, notamment sur une table d'opération. De plus, elle fournit un deuxième moyen de serrage du gilet sur le patient qui permet de tirer sur le haut de l'épaule. Comme celui qui est assuré par le quatrième panneau 4, ce serrage se réalise du côté opposé à l'épaule malade.

En général, c'est à l'hôpital que les premier et troisième panneaux 1 et 3 sont assemblés au niveau de la première courbe 11 par l'intermédiaire des moyens de fixation et de serrage 26. Le serrage est ainsi bien réglé et les moyens de fixation pourront être maintenus en position, lors de l'utilisation du gilet.

Les moyens de fixation et de serrage 26 sont similaires à ceux qui ont été précédemment décrits en référence aux figures 1 à 6.

Parmi d'autres variantes de réalisation, on peut modifier la hauteur du troisième panneau 3 et notamment le rallonger par rapport au gilet illustré sur les différentes figures. La hauteur de ce panneau est choisie pour éviter que le gilet ne remonte, lorsque le patient est allongé.

De même, une patte supplémentaire peut être prévue pour le soutien du coude.

La description précédente montre que le gilet selon l'invention peut être mis en place par un patient seul, sans aide extérieure et sans apprentissage d'une technique compliquée. Ce gilet se passe aisément par la tête et s'ajuste facilement sur les deux épaules. Il assure donc l'autonomie du patient qui peut notamment réaliser des soins d'hygiène et remettre le gilet sans aide. Ce gilet facilite également la rééducation du patient.

En particulier, le patient n'est pas obligé d'enlever complètement le gilet pour des soins d'hygiène ou une rééducation. Il lui suffit pour cela de libérer le quatrième panneau 4, en le détachant du premier panneau 1. Les premier et troisième panneaux 1 et 3 ne sont alors plus appliqués et serrés contre le corps du patient mais sont maintenus en position sur les deux épaules. De façon générale, le gilet selon l'invention est donc facilement utilisé par des personnes âgées, pour lesquelles les fractures de la tête humérale sont fréquentes.

On note encore que la fixation et le mode de réglage du gilet s'effectuent du côté opposé à la zone de traumatisme, évitant ainsi les risques de mobiliser le ou les foyers de fracture de façon intempestive.

De plus, le gilet n'enserre pas le thorax, il évite ainsi les plis désagréables et les problèmes liés à la sudation.

Comme le serrage est effectué sur la partie antérieure du thorax, la cage thoracique n'est pas comprimée, ce qui assure un meilleur confort respiratoire pour le patient.

Le coude n'est jamais enserré mais au contraire toujours à l'air libre, ce qui permet d'exercer une surveillance locale visuelle et d'effectuer des prises de pulsation sans porter atteinte à l'ensemble du dispositif.

On constate également que la partie antérieure et la partie postérieure du gilet, c'est-à-dire les premier et troisième panneaux 1 et 3, sont réunis au niveau des deux épaules et recouvrent une partie de l'humérus (droit ou gauche selon l'utilisation), ce qui a pour effet une meilleure répartition des charges et de pressions exercées sur les deux épaules.

Le gilet ne comporte qu'une seule épaisseur de tissu, ce qui est plus confortable lorsque le patient est alité.

Le fait de prévoir des moyens de serrage et de fixation des deux côtés du gilet permet d'obtenir un gilet réversible qui peut indifféremment être utilisé à gauche ou à droite.

Enfin, la languette prévue pour le soutien du pouce contribue au confort du malade.

Le gilet peut en outre être utilisé, en fin de rééducation ou de traitement orthopédique, sous la forme d'une simple écharpe de soutien de bras. Pour cela, le quatrième panneau n'est pas fixé sur la partie antérieure du gilet comme illustré à la figure 4, mais il est retourné et fixé sur la partie postérieure du gilet, c'est-à-dire sur le troisième panneau 3. Ce mode d'utilisation n'est pas illustré sur les figures.

Les signes de référence insérés après les caractéristiques techniques mentionnées dans les revendications, ont pour seul but de faciliter la compréhension de ces dernières, et n'en limitent aucunement la portée.

## Revendications

1. Gilet orthopédique de soutien et de contention de l'épaule, de la ceinture scapulaire et du membre supérieur d'un patient, ledit gilet fermant sur l'avant et étant formé de quatre panneaux :
- un premier panneau (1) antérieur recouvrant la partie antérieure du thorax et des deux épaules et la partie antérieure du bras à soutenir, ledit premier panneau se prolongeant à l'extrémité opposée aux épaules par un deuxième panneau (2) antérieur destiné à servir de reposoir à l'avant bras à soutenir, le premier panneau (1) étant délimité par une ligne (10) sensiblement rectiligne qui se raccorde au deuxième panneau (2) et qui se prolonge, du côté opposé au deuxième panneau et de façon successive, par une première courbe (11) destinée à être placée sur l'épaule et une deuxième courbe concave (12) au niveau d'une ouverture (14) ménagée entre le premier et un troisième panneau (1, 3) pour le passage de la tête du patient, ladite deuxième courbe (12) se prolonge par une troisième courbe (13) convexe descendante qui se raccorde au deuxième panneau, à l'opposé de ladite ligne (10);
un troisième panneau (3) postérieur recouvrant la partie postérieure du thorax et des deux épaules et la partie postérieure du bras à soutenir, ledit troisième panneau se prolongeant à l'extrémité opposée aux épaules du côté opposé au bras à soutenir par un quatrième panneau (4) destiné à assurer une contention et une fermeture sur l'avant dudit gilet ;
le premier panneau (1) et le troisième panneau (3) se rejoignant au niveau des épaules et le long du bras à soutenir.

2. Gilet selon la revendication 1, caractérisé en ce qu'il est ouvert au niveau de ladite première courbe (11), des moyens de serrage et de fixation (26) étant prévus pour assembler lesdits premier et troisième panneau (1, 3).

3. Gilet selon la revendication 2, caractérisé en ce que les moyens de serrage et de fixation (26) sont portés par une patte (25) qui est dans le prolongement du premier panneau (1) ou du troisième panneau (3).

4. Gilet selon l'une des revendications 1 à 3, caractérisé en ce que ledit deuxième panneau (2) présente une forme générale en demi-lune.

5. Gilet selon l'une des revendications 1 à 4, caractérisé en ce que ledit deuxième panneau (2) porte, à son extrémité libre, des moyens de serrage et de fixation (20).

6. Gilet selon l'une des revendications 4 ou 5, caractérisé en ce que le deuxième panneau (2) comporte latéralement et du côté opposé au bras à soutenir, une courbe concave (8) permettant le passage de la main du patient hors du gilet.

7. Gilet selon l'une des revendications 4 à 6, caractérisé en ce que le deuxième panneau (2) comporte latéralement et du côté du bras à soutenir, une courbe convexe (9) permettant de laisser libre le coude du patient.

8. Gilet selon l'une des revendications 1 à 7, caractérisé en ce que les premier et troisième panneaux (1 et 3) sont solidaires au niveau de la troisième courbe (13).

9. Gilet selon l'une des revendications 1 à 8, caractérisé en ce que le troisième panneau comporte une courbe concave (15), en regard de la deuxième courbe concave (12), lesdites courbes (12 et 15) définissant l'ouverture (14).

10. Gilet selon l'une des revendications 1 à 9, caractérisé en ce que le quatrième panneau (4) présente une forme allongée, sensiblement rectangulaire et comporte, à son extrémité libre, des moyens de serrage et de fixation (21).

11. Gilet selon l'une des revendications 2, 3, 5 ou 10, caractérisé en ce que lesdits moyens de serrage et de fixation (26,20,21) sont prévus sur les deux faces du gilet, de façon à ce qu'il soit réversible.

12. Gilet selon l'une des revendications 1 à 11, caractérisé en ce qu'il comporte au moins une bande amovible (5) de forme allongée, sensiblement rectangulaire, qui est destinée à allonger le quatrième panneau (4) et/ou à être fixée sur les premier (1) et troisième (3) panneaux, avant la mise en place du deuxième panneau et sous le bras malade et/ou une fois le gilet mis en place, à être fixé sur les deuxième (2) et troisième (3) panneaux et sur le bras malade.

13. Gilet selon la revendication 12, caractérisé en ce que ladite bande amovible (5) comporte, à deux extrémités libres opposées, des moyens d'attache (22, 23).

14. Gilet selon l'une des revendications 1 à 13, caractérisé en ce qu'il comporte également une languette (6) amovible pour le soutien du pouce qui forme une boucle et comporte des moyens d'attache (24), ladite languette étant destinée à être fixée sur ledit deuxième panneau (2).

15. Gilet selon l'une des revendications 1 à 14 caractérisé en ce que le gilet est constitué en une matière auto-agrippante.

16. Gilet selon l'une des revendications 1 à 14 caractérisé en ce qu'il comporte des moyens destinés à coopérer avec lesdits moyens de serrage et de fixation (26, 20, 21) et lesdits moyens d'attache (22, 23 et 24).

## Claims

1. An orthopaedic supporting vest for the shoulder, the girdle shoulder and the upper member of a patient, whereas the said vest can be closed on the front and consists of four panels:
- a first front panel (1) covering the front section of the thorax and of both shoulders as well as the front section of the arm to be supported, whereby the said first panel is extended at the end opposite to the shoulders by a second front panel (2) intended as a rest for the fore-arm to be supported, whereas the first panel (1) is delineated by a segment (10) more or less rectilinear which connects to the second panel (2) and which is extended, on the side opposite to the second panel and in succession, by a first curve (11) intended to be placed on the shoulder and a second curve (12), concave, at one opening (14) provided between the first and a third panel (1,3) for the head of the patient, whereas the said second curve (12) is extended by a third curve (13), convex descending, which connects to the second panel, opposite to the said segment (10);
a third panel (3), rear, covering the rear section of the thorax and of both shoulders as well as the rear section of the arm to be supported, whereas the said third panel is extended at the end opposite to the shoulders on the side opposite to the arm to be supported, by a fourth panel (4) intended for providing support and closing on the front of the said vest;
whereby the first panel (1) and the third panel (3) connect together at the shoulders and along the arm to be supported.

2. A vest according to claim 1, characterised in that it is open at the said first curve (11), whereas tightening and fastening means (26) are provided for the assembly of the said first and third panels (1, 3).

3. A vest according to claim 2, characterised in that the tightening and fastening means (26) are carried by a tab (25) in the extension of the first panel (1) or of the third panel (3).

4. A vest according to one of the claims 1 to 3, characterised in that the said second panel (2) is generally semi-crescent-shaped.

5. A vest according to one of the claims 1 to 4, characterised in that the said second panel (2) carries, at its free end, tightening and fastening means (20).

6. A vest according to one of the claims 4 or 5, characterised in that the second panel (2) comprises laterally and on the side opposite to the arm to be supported, a concave curve (8) enabling the patient to pass his hand through the vest.

7. A vest according to one of the claims 4 to 6, characterised in that the second panel (2) comprises laterally and on the side opposite to the arm to be supported, a convex curve (9) leaving the patient's elbow free.

8. A vest according to one of the claims 1 to 7, characterised in that the first and third panels (1 and 3) are interconnected at the third curve (13).

9. A vest according to one of the claims 1 to 8, characterised in that the third panel comprises a concave curve (15) opposite to the second concave curve (12), whereas the said curves (12 and 15) delineate the opening (14).

10. A vest according to one of the claims 1 to 9, characterised in that the fourth panel (4) is oblong, more or less rectangular and comprises, at its free end, tightening and fastening means (21).

11. A vest according to one of the claims 2, 3, 5 or 10, characterised in that the said tightening or fastening means (26, 20, 21) are provided on both faces of the vest, to make it reversible.

12. A vest according to one of the claims 1 to 11, characterised in that it comprises at least one oblong removable band (5), more or less rectangular, intended to extend the fourth panel (4) and/or to be fastened on the first (1) and third (3) panels, before placing the second panel and under the suffering arm and/or after placing the vest, to be fastened on the second (2) and third (3) panels and on the suffering arm.

13. A vest according to claim 12, characterised in that the said removable band (5) comprises, at two free opposite ends, attachment means (22, 23).

14. A vest according to one of the claims 1 to 13, characterised in that it also comprises a removable tongue (6) for supporting the thumb which forms a loop and comprises attachment means (24), whereas the said tongue is intended for fastening on the said second panel (2).

15. A vest according to one of the claims 1 to 14, characterised in that the vest consists of a self-adhesive material.

16. A vest according to one of the claims 1 to 14, characterised in that it comprises means intended for co-operating with the said tightening and fastening leans (26, 20, 21) and the said attachment means (22, 23 and 24).

## Patentansprüche

1. Orthopädische Weste zur Unterstützung und Ruhigstellung der Schulter, des Schultergürtels und der oberen Gliedmaße eines Patienten, wobei die Weste vorne zu schließen und aus vier Flächenteilen gebildet ist:
- einem ersten, vorderen Flächenteil (1), der den vorderen Teil des Brustkorbs und der beiden Schultern und den vorderen Teil des zu unterstützenden Armes bedeckt, wobei der erste Flächenteil an dem Ende, das den beiden Schultern gegenüberliegt, durch einen zweiten Flächenteil (2) verlängert ist, der dazu bestimmt ist, als Ruhestütze für den zu unterstützenden Unterarm zu dienen,
wobei der erste Flächenteil (1) durch eine im wesentlichen gerade Linie (10) begrenzt ist, die sich mit dem zweiten Flächenteil (2) verbindet und die sich von der dem zweiten, entgegengesetzten Flächenteil her und in ununterbrochener Weise in einer ersten Krümmung (11), die dazu bestimmt ist, auf die Schulter gelegt zu werden, und einer zweiten, konkaven Krümmung (12) auf Höhe einer Öffnung (14) fortsetzt, die zwischen dem ersten Flächenteil und einem dritten Flächenteil (1, 3) für das Durchlassen des Kopfes des Patienten vorgesehen ist, wobei sich die zweite Krümmung (12) in einer dritten, abfallend konvexen Krümmung (13) fortsetzt, die sich mit dem zweiten Flächenteil gegenüber der Linie (10) verbindet,
- einem dritten, hinteren Flächenteil (3) zum Bedecken des hinteren Teiles des Brustkorbes und der beiden Schultern und des hinteren Teiles des zu unterstützenden Armes, wobei sich der dritte Flächenteil an dem Ende, das den Schultern auf der Seite, die dem zu unterstützenden Arm gegenüberliegt, durch einen vierten Flächenteil (4) fortsetzt, der dazu bestimmt ist, eine Ruhigstellung und einen Verschluß auf der Vorderseite der Weste sicherzustellen,
wobei der erste Flächenteil (1) und der dritte Flächenteil (3) in Höhe der Schultern und auf der Länge des zu unterstützenden Armes aneinanderfügbar sind.

2. Weste nach Anspruch 1, dadurch gekennzeichnet, daß sie bei der ersten Krümmung (11) offen ist, wobei Spann- und Befestigungsmittel (26) zum Aneinanderfügen des ersten und dritten Flächenteils (1, 3) vorgesehen sind.

3. Weste nach Anspruch 2, dadurch gekennzeichnet, daß die Spann- und Befestigungsmittel (26) von einer Lasche (25) getragen werden, die sich in der Verlängerung des ersten Flächenteils (1) oder des dritten Flächenteils (3) befindet.

4. Weste nach einem der Ansprüche 1 bis 3, dadurch gekennzeichnet, daß der zweite Flächenteil (2) eine allgemeine Halbmondform zeigt.

5. Weste nach einem der Ansprüche 1 bis dadurch gekennzeichnet, daß der zweite Flächenteil (2) an seinem freien Ende Spann- und Befestigungsmittel (20) trägt.

6. Weste nach einem der Ansprüche 4 oder 5, dadurch gekennzeichnet, daß der zweite Flächenteil (2) seitlich und von der dem zu unterstützenden Arm entgegengesetzten Seite her eine konkave Krümmung (8) aufweist, die das Hindurchgehen der Hand des Patienten aus der Weste heraus gestattet.

7. Weste nach einem der Ansprüche 4 bis 6, dadurch gekennzeichnet, daß der zweite Flächenteil (2) seitlich und von der Seite des zu unterstützenden Armes her eine konvexe Krümmung (9) aufweist, die es gestattet, den Ellbogen des Patienten frei zu lassen.

8. Weste nach einem der Ansprüche 1 bis 7, dadurch gekennzeichnet, daß der erste und dritte Flächenteil (1 und 3) bei der dritten Krümmung (13) fest miteinander verbunden sind.

9. Weste nach einem der Ansprüche 1 bis 8, dadurch gekennzeichnet, daß der dritte Flächenteil eine der zweiten konkaven Krümmung (12) gegenüberstehende konkave Krümmung (15) aufweist, wobei die Krümmungen (12 und 15) die Öffnung (14) definieren.

10. Weste nach einem der Ansprüche 1 bis 9, dadurch gekennzeichnet, daß der vierte Flächenteil (4) eine längliche, im wesentlichen rechteckige Form aufweist und an seinem freien Ende Spann- und Befestigungsmittel (21) aufweist.

11. Weste nach einem der Ansprüche 2, 3, 5 oder 10, dadurch gekennzeichnet, daß die Spann- und Befestigungsmittel (26, 20, 21) auf den beiden Seiten der Weste derart vorgesehen sind, daß sie beiderseitig tragbar ist.

12. Weste nach einem der Ansprüche 1 bis 11, dadurch gekennzeichnet, daß sie wenigstens einen lösbaren Streifen (5) mit länglicher, im wesentlichen rechteckiger Form aufweist, der dazu bestimmt ist, den vierten Flächenteil (4) zu verlängern und/oder an dem ersten (1) und dritten (3) Flächenteil vor dem Anlegen des zweiten Flächenteiles und unter dem kranken Arm befestigt zu werden, und/oder wenn die Weste einmal angelegt ist, an dem zweiten (2) und dritten (3) Flächenteil auf dem kranken Arm befestigt zu werden.

13. Weste nach Anspruch 12, dadurch gekennzeichnet, daß der lösbare Streifen (5) an zwei entgegengesetzten freien Enden Befestigungsmittel (22, 23) aufweist.

14. Weste nach einem der Ansprüche 1 bis 13, dadurch gekennzeichnet, daß sie ebenfalls eine lösbare Lasche (6) zur Unterstützung des Daumens aufweist, die eine Schlaufe bildet und Befestigungsmittel (24) aufweist, wobei die Lasche dazu bestimmt ist, an dem zweiten Flächenteil (2) befestigt zu werden.

15. Weste nach einem der Ansprüche 1 bis 14, dadurch gekennzeichnet, daß die Weste aus einem selbst festhaltenden Material besteht.

16. Weste nach einem der Ansprüche 1 bis 14, dadurch gekennzeichnet, daß sie Mittel aufweist, die dazu bestimmt sind mit den Spann- und Befestigungsmitteln (26, 20, 21) und den Befestigungsmitteln (22, 23 und 24) zusammenzuwirken.
